(19) Europäisches Patentamt
European Patent Office
Office européen des brevets

(11) Veröffentlichungsnummer: **0 305 804 B1**

# (12) EUROPÄISCHE PATENTSCHRIFT

(45) Veröffentlichungstag der Patentschrift: **16.09.92**

(51) Int. Cl.$^5$: **C08G 77/46**, C08G 18/61, C08L 101/00

(21) Anmeldenummer: **88113323.5**

(22) Anmeldetag: **17.08.88**

## (54) Wasserhärtende Polymerzubereitung.

(30) Priorität: **26.08.87 DE 3728396**
**15.04.88 DE 3812481**

(43) Veröffentlichungstag der Anmeldung:
**08.03.89 Patentblatt 89/10**

(45) Bekanntmachung des Hinweises auf die Patenterteilung:
**16.09.92 Patentblatt 92/38**

(84) Benannte Vertragsstaaten:
**AT BE CH DE ES FR GB GR IT LI LU NL SE**

(56) Entgegenhaltungen:
**EP-A- 0 083 733**
**WO-A-85/05322**
**DE-A- 2 357 931**
**US-A- 4 163 830**

(73) Patentinhaber: **BAYER AG**

**W-5090 Leverkusen 1 Bayerwerk(DE)**

(72) Erfinder: **Jansen, Bernhard, Dr.**
**Roggendorfstrasse 65**
**W-5000 Köln 80(DE)**
Erfinder: **Müller, Hanns Peter, Dr.**
**Weizenfeld 36**
**W-5060 Bergisch Gladbach 2(DE)**
Erfinder: **Richter, Roland, Dr.**
**Hermann-Ehlert-Strasse 12**
**W-5090 Leverkusen(DE)**
Erfinder: **Mayer, Wolfram, Dr.**
**Auf dem Heidchen 17**
**W-5068 Odenthal-Gloebusch(DE)**

EP 0 305 804 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Konstruktionsmaterial, bestehend aus einer Trägerschicht, die mit einer wasserhärtenden Polymerzubereitung beschichtet und/oder imprägniert ist, wobei die Polymerzubereitung als Additiv Polyether-polysiloxan-Polyurethane enthält, ein Verfahren zu ihrer Herstellung und ihre Verwendung.

Die erfindungsgemäßen Konstruktionsmaterialien werden insbesondere für medizinische Stützverbände oder technische Vorrichtungen verwendet.

Im allgemeinen können die erfindungsgemäßen Konstruktionsmaterialien zur Versteifung, Formgebung und Abdichtung im medizinischen oder technischen Bereich verwendet werden. Im medizinischen Bereich werden sie bevorzugt als Gipsersatz eingesetzt.

Die erfindungsgemäßen Konstruktionsmaterialien Können aber auch zur Herstellung von Behältern, Filtern, von Rohren, zum Verbinden von Konstruktionselementen, zur Fabrikation von dekorativen oder künstlerischen Artikeln, zu Versteifungszwecken oder als Füll- bzw. Dichtungsmaterial für Fugen und Hohlräume eingesetzt werden.

Die zur Herstellung der erfindungsgemäßen Konstruktionsmaterialien eingesetzten wasserhärtenden Polymerzubereitungen sind aus der US-PS 4 163 830 bereits bekanntgeworden. Gemäß diesem Dokument sind sie Zwischenprodukte zur Herstellung verzweigter Polysiloxan-Polyoxyalkylen-Copolymere, die zur Herstellung von Polyurethanschäumen mit verbessertem Brandverhalten eingesetzt werden. Zu ihrer erfindungsgemäßen Eignung zur Herstellung von z.B. orthopädischen Stützverbänden gibt der Stand der Technik keinerlei Hinweise.

Konstruktionsmaterialien, die aus einem flexiblen Träger bestehen, der mit einem wasserhärtenden Reaktivharz beschichtet und/oder getränkt ist, sind bereits bekannt. Beispielsweise sei die DE-A-23 57 931 genannt, in der Konstruktionsmaterialien aus flexiblen Trägern, wie Gewirken, Geweben oder Vliesen, beschrieben werden, die mit wasserhärtenden Reaktivharzen, wie Isocyanaten oder durch Isocyanatgruppen modifizierte Prepolymere, beschichtet oder getränkt sind.

Die aus der DE-A-23 57 931 bekannten wasserhärtenden Polymerzubereitungen und die späteren Nachfolgeentwicklungen haben den Nachteil, daß sie sich nur schlecht modellieren lassen, insbesondere während der Aushärtungsphase, da sie dann eine starke Klebrigkeit entwickeln.

Dieses Problem versucht man in der EP-A-02 21 669 durch Zusatz von Schmiermitteln zu lösen. Die in der EP-A-02 21 669 beschriebenen Flächengebilde, z.B. orthopädische Binden, bestehen aus einem mit einer wasserhärtenden Polymerzubereitung beschichteten Träger, der oberflächlich zum größten Teil mit einem Schmiermittel beschichtet ist. Bei den Schmiermitteln handelt es sich um Verbindungen mit hydrophilen Gruppen, die kovalent an das Polymer gebunden sind oder um Additive die inkompatibel mit dem Polymer sind. Die Menge an Schmiermittel wird so hoch gewählt, daß sie einen kinetischen Reibungkoeffizienten der beschichteten Flächengebilde von weniger als 1,2 bewirken. Die so behandelten Flächengebilde weisen eine geringe Klebrigkeit auf. Als unverträgliche, nicht mischbare Additive werden unter anderem auch Polysiloxane eingesetzt (Seite 8, Zeile 28 bis Seite 10, Zeile 8).

Bei den bekannten Materialien ist es für eine deutliche Reduzierung des kinetischen Reibungskoeffizienten (z.B. unter 1,2) notwendig, daß ein Schmiermittel vorhanden ist, welches hydrophile Gruppen aufweist, die an das Polymer gebunden sind, und/oder ein Additiv enthalten ist, wie beispielsweise die genannten Polysiloxane, welche mit der Polymerzubereitung nicht mischbar ist, also separat aufgebracht werden müssen.

Die erfindungsgemäßen Konstruktionsmaterialien enthalten wasserhärtende Polymerzubereitungen, die als Additive Polymermodifikatoren vom Typ der Polyetherpolysiloxan-Polyurethane der Formel

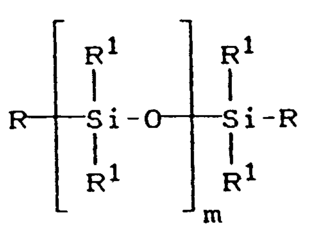 (I),

in der

- $R^1$ für Niederalkyl steht,
- m für die mittlere Zahl an Siloxangruppen im Bereich von 5 bis 25 steht,
- R für den Rest

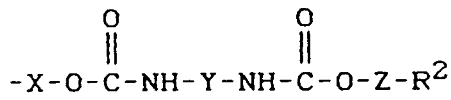

steht,
worin

- X für einen Niederalkylenrest steht,
- Y für einen aliphatischen, cycloaliphatischen, aromatischen oder araliphatischen Rest steht,
- Z für einen Polyetherrest mit Ethylenoxid- und/oder Propylenoxid-Gruppen steht, wobei die mittlere Zahl der Ethylenoxid und/oder Propylenoxid-Gruppen im Bereich von 5 bis 150 liegt, und
- $R^2$ für Niederalkyl steht,

wobei

- Y durch weitere

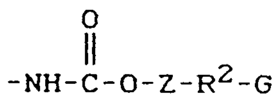
-Gruppen

substituiert sein kann,
in denen

- Z und $R^2$ die obengenannte Bedeutung haben,

enthalten.

Die erfindungsgemäßen Additive sind kompatibel mit den Polymerzubereitungen und vermischen sich völlig mit diesen. Sie zeigen eine ausgezeichnete Lagerstabilität und entmischen sich auch nach mehr als 12 Monaten Lagerung nicht.

Der Vorteil der erfindungsgemäßen Systeme liegt darin, daß man sie bereits bei der Herstellung der Reaktivharze beimischen kann, was gegenüber bekannten Materialien einen Verarbeitungsschritt einspart.

Die Zubereitungen lassen sich während der Aushärtzeit leicht modellieren, jedoch sind sie während der Verarbeitungsphase nicht so schlüpfrig, wie die Verbindungen der EP-A 221 669, wo das Gleitmittel als separate Schicht aufgebracht ist. Die erfindungsgemäßen Zubereitungen entwickeln ihre gute Modellierbarkeit erst nach dem Benetzen mit Wasser, wodurch eine bequeme Verarbeitung ermöglicht wird. Der kinetische Reibungskoeffizient liegt dann ebenfalls unter 1,2, was die geringe Klebrigkeit verdeutlicht.

Im Rahmen der vorliegenden Erfindung können im allgemeinen die Substituenten der Polyetherpolysiloxan-Polyurethane die folgende Bedeutung haben:
Niederalkyl kann im allgemeinen einen geradkettigen oder verzweigten Kohlenwasserstoffrest mit 1 bis etwa 6 Kohlenstoffatomen bedeuten. Beispielsweise seien Methyl, Ethyl, Propyl, Isopropyl, Butyl, Isobutyl, Pentyl, Isopentyl, Hexyl und Isohexyl genannt. Bevorzugt werden Methyl und Ethyl. Insbesondere bevorzugt wird Methyl.

Niederalkylen kann im allgemeinen einen zweibindigen, geradkettigen oder verzweigten Kohlenwasserstoffrest mit 1 bis etwa 6 Kohlenstoffatomen bedeuten. Beispielsweise sei Methylen, Ethylen, Propylen, Isopropylen, Butylen, Isobutylen, Pentylen, Isopentylen, Hexylen und Isohexylen genannt. Bevorzugt wird Methylen, Ethylen und Propylen.

Ein aliphatischer Rest kann im allgemeinen für einen geradkettigen oder verzweigten aliphatischen Kohlenwasserstoffrest mit 2 bis 18, vorzugsweise 6 bis 12, Kohlenstoffatomen stehen. Beispielsweise seien die folgenden Reste genannt:

$-(CH_2)_6-$
$-(CH_2)_{12}-$

Vorzugsweise sei $-(CH_2)_6-$ genannt.

Ein cycloaliphatischer Rest kann im allgemeinen ein cycloaliphatischer Kohlenwasserstoffrest mit 4 bis 15, vorzugsweise 5 bis 10, Kohlenstoffatomen sein. Beispielsweise seien die folgenden cycloaliphatischen Kohlenwasserstoffreste genannt:

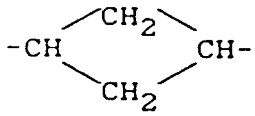 , 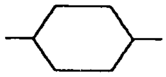 , 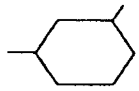 , 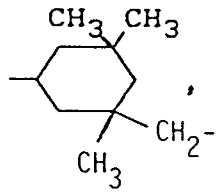 ,

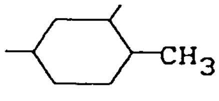 , 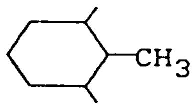

Bevorzugt sind:

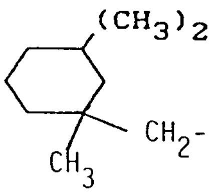 , 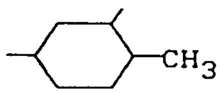 und 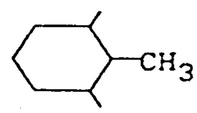

Ein aromatischer Rest kann im allgemeinen ein aromatischer Kohlenwasserstoffrest mit 6 bis 15, vorzugsweise 6 bis 13, Kohlenstoffatomen sein. Als aromatische Reste seien beispielsweise genannt: Phenyl, Naphthyl und Biphenyl. Bevorzugt ist Phenyl.

Ein araliphatischer Rest kann im allgemeinen ein araliphatischer Kohlenwasserstoffrest mit 8 bis 15, vorzugsweise 8 bis 13, Kohlenstoffatomen sein Als araliphatische Reste seien beispielsweise genannt: Benzyl,

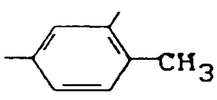 , 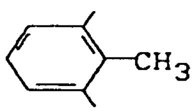 , 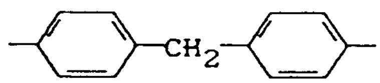 ,

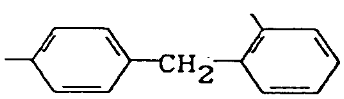 , 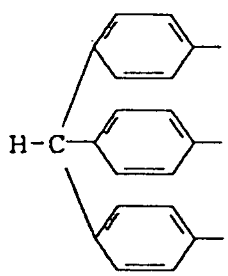 ,

Bevorzugt sind:

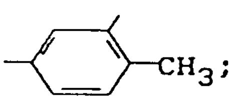 ; 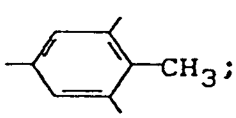 ; 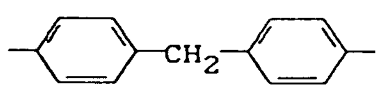

Die genannten aliphatischen, cycloaliphatischen, aromatischen und araliphatischen Reste (Y) können gegebenenfalls durch weitere, bevorzugt durch zwei, insbesondere bevorzugt durch einen, weiteren Substituenten der Formel

$$-NH-\overset{\overset{O}{\|}}{C}-O-Z-R^2$$

substituiert sein.

Für Y seien im einzelnen beispielsweise Reste genannt, die sich von den folgenden niedermolekularen Polyisocyanaten ableiten:
Geeignete derartige niedermolekulare Polyisocyanate sind z.B. Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethylcyclohexan, 2,4- und 2,6-Hexahydrotoluylendiisocyanat sowie beliebige Gemische dieser Isomeren, Hexahydro-1,3- und/oder -1,4-phenylendiisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4',4"-triisocyanat oder Polyphenyl-polymethylenpolyisocyanate, wie sie durch Anilin-Formaldehyd-Kondensation und anschließende Phosgenierung erhalten werden.

Geeignete höhermolekulare Polyisocyanate sind Modifizierungsprodukte derartiger einfacher Polyisocyanate, d.h. Polyisocyanate mit z.B. Isocyanurat-, Carbodiimid-, Allophanat-, Biuret- oder Uretdion-Struktureinheiten, wie sie nach an sich bekannten Verfahren des Standes der Technik aus den beispielhaft genannten einfachen Polyisocyanaten der obengenannten allgemeinen Formel hergestellt werden können.

Erfindungsgemäß besonders bevorzugte Polyisocyanatkomponenten sind die in der Polyurethanchemie üblichen technischen Polyisocyanate, d.h. Hexamethylendiisocyanat, 2,4- und 2,6-Toluylendiisocyanat, 1-Isocyanato-3,3,5-trimethyl-5-isocyanatomethyl-cyclohexan (Isophorondiisocyanat, abgekürzt: IPDI), 4,4'-Diisocyanato-dicyclohexylmethan, 4,4'-Diisocyanatodiphenylmethan, dessen Gemische mit den entsprechenden 2,4'- und 2,2'-Isomeren, Polyisocyanatgemische der Diphenylmethanreihe wie sie durch Phosgenierung von Anilin/Formaldehyd-Kondensaten in an sich bekannter Weise gewonnen werden können und die Biuret- oder Isocyanuratgruppen aufweisenden Modifizierungsprodukte dieser technischen Polyisocyanate sowie beliebige Gemische derartiger Polyisocyanate. Isocyanate mit aromatisch gebundenen NCO-Gruppen sind erfindungsgemäß bevorzugt. Eine erfindungsgemäß besonders bevorzugte Polyisocyanat-Komponente stellen 2,4-und 2,6-Toluylendiisocyanat und/oder Gemische aus den beiden Isomeren dar.

Der Polyetherrest (Z) besteht im allgemeinen aus Ethylenoxid- und/oder Propylenoxid-Gruppen. Bevorzugt besteht er aus Ethylenoxid- und Propylenoxid-Gruppen. Die mittlere Gesamtzahl der Ethylenoxid- und Propylen-Gruppen liegt im allgemeinen im Bereich von etwa 5 bis etwa 150, bevorzugt von 10 bis 100. Für den bevorzugten Fall, daß der Polyetherrest aus Ethylenoxid- und Propylenoxid-Gruppen besteht, beträgt im allgemeinen das Gew.-Verhältnis Ethylenoxid zu Propylenoxid 10:90 % bis 80:20 %, bevorzugt 20:80 % bis 75:25 %.

Die Ethylenoxid- und Propylenoxid-Gruppen können statistisch, alternierend oder in Blöcken geordnet sein. Bevorzugt ist eine statistische Verteilung.

Bevorzugt können die erfindungsgemäß verwendeten wasserhärtenden Polymerzubereitungen als Additive Polyetherpolysiloxan-Polyurethane der Formel (I) enthalten,
in der

$R^1$ für Methyl oder Ethyl steht,
m für die mittlere Zahl an Siloxangruppen im Bereich von 5 bis 25 steht,
R für den Rest

$$-X-O-\overset{\overset{O}{\|}}{C}-NH-Y-NH-\overset{\overset{O}{\|}}{C}-O-Z-R^2$$

steht,
worin
X für Alkylen ($C_1$ bis $C_4$) steht,
Y einen aliphatischen Kohlenwasserstoffrest mit 2 bis 18 Kohlenstoffatomen,
einen cycloaliphatischen Kohlenwasserstoffrest mit 4 bis 15 Kohlenstoffatomen,

einen aromatischen Kohlenwasserstoffrest mit 6 bis 15 Kohlenstoffatomen oder
einen araliphatischen Kohlenwasserstoffrest mit 8 bis 15 Kohlenstoffatomen bedeutet,

Z für einen Polyetherrest mit Ethylenoxid- und Propylenoxid-Gruppen steht, wobei die mittlere Zahl der Ethylenoxid- und Propylenoxid-Gruppen im Bereich von 10 bis 100 liegt, und

$R^2$ für Alkyl ($C_1$ bis $C_4$) steht,
wobei das Gewichtsverhältnis der Ethylenoxid- zu Propylenoxid-Gruppen 10:90 % bis 80:20 % beträgt und
wobei Y durch 1 oder 2 weitere

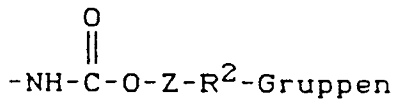

substituiert sein kann,
in denen

Z und $R^2$ die obengenannte Bedeutung haben.

Insbesondere bevorzugt können die erfindungsgemäß verwendeten wasserhärtenden Polymerzubereitungen als Additive Polyetherpolysiloxan-Polyurethane der Formel (I) enthalten
in der

$R^1$ für Methyl oder Ethyl steht,

m für die mittlere Zahl an Siloxangruppen im Bereich von 5 bis 25 steht,

R für den Rest

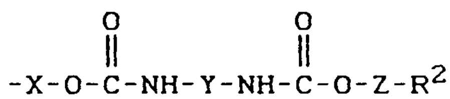

steht,
worin

X für Alkylen ($C_1$ bis $C_4$) steht,

Y einen aliphatischen Kohlenwasserstoffrest mit 6 bis 10 Kohlenstoffatomen,
einen cycloaliphatischen Kohlenwasserstoffrest mit 5 bis 10 Kohlenstoffatomen,
einen aromatischen Kohlenwasserstoffrest mit 6 bis 13 Kohlenstoffatomen oder
einen araliphatischen Kohlenwasserstoffrest mit 8 bis 13 Kohlenstoffatomen bedeutet,

Z für einen Polyetherrest mit Ethylenoxid- und Propylenoxid-Gruppen steht, wobei die mittlere Zahl der Ethylenoxid- und Propylenoxid-Gruppen im Bereich von 10 bis 100 liegt, und

$R^2$ für Alkyl ($C_1$ bis $C_4$) steht,
wobei das Gewichtsverhältnis der Ethylenoxid- zu Propylenoxid-Gruppen 10:90 % bis 80:20 % beträgt und
wobei Y durch eine weitere

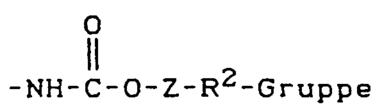

substituiert sein kann,
in der

Z und $R^2$ die obengenannte Bedeutung haben.

Die Gruppe der als Additive verwendeten Polyetherpolysiloxan-Polyurethane ist an sich bekannt (DE-A-25 58 523, US 4.096.162); sie werden als Stabilisatoren für Polyurethanschäume eingesetzt.

Die Polyetherpolysiloxan-Polyurethane können hergestellt werden, indem man im Isocyanat OCN-Y-NCO mit einem monohydroxyfunktionellen Polyether HO-Z-$R^2$ und dann das Reaktionsprodukt OCN-Y-NH-CO-O-Z-$R^2$ mit einem Bishydroxyalkyldialkylpolysiloxan

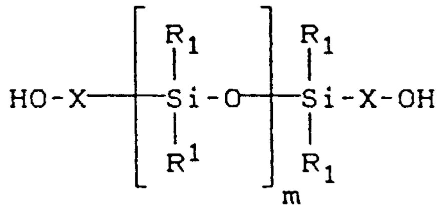

wobei
X,Y,Z,$R^1$,$R^2$ und m die obengenannte Bedeutung haben,
umsetzt.

Die erfindungsgemäß verwendeten wasserhärtenden Polymerzubereitungen enthalten im allgemeinen 0,1 bis 10 Gew,-%, bevorzugt 0,5 bis 8 Gew.-%, Polyetherpolysiloxan-Polyurethane, bezogen auf das Polymerharz, wobei der kinetische Reibungskoeffizient um 0,5 oder mehr kleiner sein soll als der ohne die Menge der entsprechenden Additive.

Selbstverständlich ist es möglich, daß die erfindungsgemäßen wasserhärtenden Polymerzubereitungen mehrerer der genannten erfindungsgemäßen Polyetherpolysiloxan-Polyurethane als Additive enthalten.

Wasserhärtende Polymerzubereitungen sind bevorzugt Harze auf Polyurethan- oder Polyvinylharz-Basis.

Besonders bevorzugt sind Polyisocyanate bzw. Prepolymere mit freien Isocyanatgruppen (Polyurethane).

Als wasserhärtende Polyisocyanate bzw. Polyurethane kommen erfindungsgemäß alle an sich bekannten organischen Polyisocyanate in Frage, d.h. beliebige Verbindungen bzw. Gemische von Verbindungen, die pro Molekül mindestens zwei organisch gebundene Isocyanatgruppen aufweisen. Hierzu gehören sowohl niedermolekulare Polyisocyanate mit einem unter 400 liegendem Molekulargewicht als auch Modifizierungsprodukte derartiger niedermolekularer Polyisocyanate mit einem aus der Funktionalität und dem Gehalt an funktionellen Gruppen berechenbaren, z.B. 400 bis 10.000, vorzugsweise 600 bis 8.000, und insbesondere 800 bis 5.000, betragenden Molekulargewicht. Geeignete niedermolekulare Polyisocyanate sind beispielsweise solche der Formel

$Q\,(NCO)_n$,

in der
n = 2 bis 4, vorzugsweise 2 bis 3,
und

Q einen aliphatischen Kohlenwasserstoffrest mit 2 bis 18, vorzugsweise 6 bis 10 C-Atomen, einen cycloaliphatischen Kohlenwasserstoffrest mit 4 bis 15, vorzugsweise 5 bis 10 C-Atomen,
einen aromatischen Kohlenwasserstoffrest mit 6 bis 15, vorzugsweise 6 bis 13 C-Atomen,
oder einen araliphatischen Kohlenwasserstoffrest mit 8 bis 15, vorzugsweise 8 bis 13 C-Atomen,

bedeuten.

Geeignete derartige niedermolekulare Polyisocyanate sind z.B. Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethylcyclohexan, 2,4- und 2,6-Hexahydrotoluylendiisocyanat sowie beliebige Gemische dieser Isomeren, Hexahydro-1,3- und/oder -1,4-phenylendiisocyanat, Perhydro-2,4'-und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4',4"-triisocyanat oder Polyphenyl-polymethylenpolyisocyanate, wie sie durch Anilin-Formaldehyd-Kondensation und anschließende Phosgenierung erhalten werden.

Geeignete höhermolekulare Polyisocyanate sind Modifizierungsprodukte derartiger einfacher Polyisocyanate, d.h. Polyisocyanate mit z.B. Isocyanurat-, Carbodiimid-, Allophanat-, Biuret- oder Uretdion-Struktureinheiten, wie sie nach an sich bekannten Verfahren des Standes der Technik aus den beispielhaft genannten einfachen Polyisocyanaten der oben genannten allgemeinen Formel hergestellt werden können. Unter den höhermolekularen, modifizierten Polyisocyanaten sind insbesondere die aus der Polyurethanchemie bekannten Prepolymeren mit endständigen Isocyanatgruppen des Molekulargewichtsbereichs 400 bis 10.000, vorzugsweise 600 bis 8.000 und insbesondere 800 bis 5.000, von Interesse. Diese Verbindungen werden in an sich bekannter Weise durch Umsetzung von überschüssigen Mengen an einfachen Polyisocyanaten der beispielhaft genannten Art mit organischen Verbindungen mit mindestens zwei gegenüber Isocyanatgruppen reaktionsfähigen Gruppen, insbesondere organischen Polyhydroxylverbindungen hergestellt. Geeignete derartige Polyhydroxylverbindungen sind sowohl einfache mehrwertige Alkohole wie z.B. Ethylenglykol, Trimethylolpropan, Propandiol-1,2 oder Butandiol-1,2, insbesondere jedoch höhermolekulare Polyetherpolyole und/oder Polyesterpolyole der aus der Polyurethanchemie an sich bekannten Art mit Molekulargewichten von 600 bis 8.000, vorzugsweise 800 bis 4.000, die mindestens zwei, in der Regel 2 bis 8, vorzugsweise aber 2 bis 4 primäre und/oder sekundäre Hydroxylgruppen aufweisen. Selbstverständlich können auch solche NCO-Prepolymere eingesetzt werden, die beispielsweise aus niedermolekularen Polyisocyanaten der beispielhaft genannten Art und weniger bevorzugten Verbindungen mit gegenüber Isocyanatgruppen reaktionsfähigen Gruppen wie z.B. Polythioetherpolyolen, Hydroxylgruppen aufweisenden Polyacetalen, Polyhydroxypolycarbonaten, Hydroxylgruppen aufweisenden Polyesteramiden oder Hydroxylgruppen aufweisenden Copolymerisaten olefinisch ungesättigter Verbindungen erhalten worden sind, Zur Herstellung der NCO-Prepolymeren geeignete Verbindungen mit gegenüber Isocyanatgruppen reaktionsfähigen Gruppen, insbesondere Hydroxylgruppen, sind beispielsweise die in US-PS 4 218 543, Kolonne 7, Zeile 29 bis Kolonne 9, Zeile 25 beispielhaft offenbarten Verbindungen. Bei der Herstellung der NCO-Prepolymeren werden diese Verbindungen mit gegenüber Isocyanatgruppen reaktionsfähigen Gruppen mit einfachen Polyisocyanaten der oben beispielhaft genannten Art unter Einhaltung eines NCO/OH-Äquivalentverhältnisses von >1 zur Umsetzung gebracht. Die NCO-Prepolymeren weisen im allgemeinen einen NCO-Gehalt von 2,5 bis 30, vorzugsweise 6 bis 25 Gew.-% auf. Hieraus geht bereits hervor, daß im Rahmen der vorliegenden Erfindung unter "NCO-Prepolymeren" bzw. unter "Prepolymeren mit endständigen Isocyanatgruppen" sowohl die Umsetzungsprodukte als solche als auch ihre Gemische mit überschüssigen Mengen an nicht umgesetzten Ausgangspolyisocyanaten, die oft auch als "Semiprepolymer" bezeichnet werden, zu verstehen sind.

Erfindungsgemäß besonders bevorzugte Polyisocyanatkomponenten sind die in der Polyurethanchemie üblichen technischen Polyisocyanate, d.h. Hexamethylendiisocyanat, 1-Isocyanato-3,3,5-trimethyl-5-isocyanatomethyl-cyclohexan (Isophorondiisocyanat, abgekürzt: IPDI), 4,4'-Diisocyanato-dicyclohexylmethan,4,4'-Diisocyanatodiphenylmethan, dessen Gemische mit den entsprechenden 2,4'- und 2,2'-Isomeren, Polyisocyanatgemische der Diphenylmethanreihe wie sie durch Phosgenierung von Anilin/Formaldehyd-Kondensaten in an sich bekannter Weise gewonnen werden können, die Biuret- oder Isocyanuratgruppen aufweisenden Modifizierungsprodukte dieser technischen Polyisocyanate und insbesondere NCO-Prepolymere der genannten Art auf Basis dieser technischen Polyisocyanate einerseits und der beispielhaft genannten einfachen Polyolen und/oder Polyetherpolyolen und/oder Polyesterpolyolen andererseits, sowie beliebige Gemische derartiger Polyisocyanate. Isocyanate mit aromatisch gebundenen NCO-Gruppen sind erfindungsgemäß bevorzugt. Eine erfindungsgemäß besonders bevorzugte Polyisocyanat-Komponente stellt teilweise carbodiimidisiertes Diisocyanatodiphenylmethan dar, welches infolge Anlagerung von monomerem Diisocyanat an die Carbodiimid-Struktur auch Uretonimingruppen aufweist.

Die wasserhärtenden Polyurethane können an sich bekannte Katalysatoren enthalten. Insbesondere können dies tert. Amine sein, die die Isocyanat/Wasser-Reaktion und nicht eine Selbstreaktion (Trimerisierung, Allophanatisierung) katalysieren (DE-A 23 57 931). Als Beispiele seien genannt tert. aminhaltige Polyether (DE-A 26 51 089), niedermolekulare tert. Amine, wie

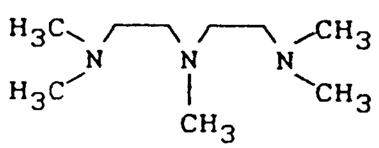

oder Dimorpholindiethylether oder Bis-(2,6-dimethylmorpholino)-diethylether (WO 86/01397). Der Gehalt an Katalysator bezogen auf den tert.-Stickstoff beträgt im allgemeinen 0,05 bis 0,5 Gew.-% bezogen auf das Polymerharz.

Wasserhärtende Polyvinylharze können beispielsweise Vinylverbindungen sein, die aus einem hydrophilen Prepolymer mit mehr als einer polymerisierbaren Vinylgruppe bestehen, in der ein fester, unlöslicher Vinyl-Redox Katalysator eingelagert ist, dessen einer Bestandteil von einer wasserlöslichen bzw. wasserdurchlässigen Hülle umkapselt ist. Ein solcher Redox Katalysator ist beispielsweise Natriumhydrogensulfit/Kupfer(II)sulfat, bei dem beispielsweise das Kupfersulfat mit Poly-2-hydroxyethylmethylacrylat verkapselt ist.

Polyvinylharze werden beispielsweise in der EP-A 01 36 021 beschrieben.

Die wasserhärtenden Polymerzubereitungen können an sich bekannte Zusatzmittel enthalten, wie z.B. Verlaufshilfsmittel, Thixotropiermittel, Entschäumer und andere bekannte Gleitmittel.

Weiterhin können die Kunststoffharze eingefärbt sein oder, falls erwünscht, UV-Stabilisatoren enthalten.

Als Zusatzmittel seien beispielsweise genannt: Polydimethylsiloxane, Calciumsilikate vom Aerosil-Typ, Polywachse (Polyethylenglykole), UV-Stabilisatoren vom Ionol-Typ (DE-A 29 21 163), Farbpigmente, wie Ruß, Eisenoxide, Titandioxid oder Phthalocyanine.

Die insbesondere für Polyurethan-Prepolymere geeigneten Zusatzmittel sind im Kunststoff-Handbuch, Band 7, Polyurethane, Seiten 100 bis 109 (1983) beschrieben. Sie werden im allgemeinen in einer Menge von 0,5 bis 5 % (bezogen auf das Harz) zugesetzt.

Als Trägermaterialien kommen massive oder poröse Folien oder auch Schaumstoffe aus natürlichen oder synthetischen Materialien (z.B. Polyurethan) in erster Linie luftdurchlässige, flexible Flächengebilde auf textiler Basis in Betracht, vorzugsweise mit einem Flächengewicht von 20 bis 1.000 g/m$^2$, insbesondere von 30 bis 500 g/m$^2$. Als Flächengebilde seien beispielsweise genannt:

Trägermaterial

1. Textile Gewebe, Gewirke oder Gestricke eines Flächengewichts von 20 bis 400 g/m$^2$, vorzugsweise 40 bis 250 g/m$^2$, mit 25 bis 100 Maschenreihen pro 10 cm, vorzugsweise 30 bis 75 Maschenreihen pro 10 cm und 30 bis 90 Maschenstäbe pro 10 cm, vorzugsweise 40 bis 80 Maschenstäbe pro 10 cm. Das textile Gewebe, Gewirke oder Gestricke kann aus beliebigen natürlichen oder synthetischen Garnen hergestellt sein. Vorzugsweise werden Garne eingesetzt die aus Baumwolle, Polyester-, Polyacrylat-, Polyamid- oder Elasthan-Fasern bestehen bzw. aus Gemischen der vorgenannten. Besonders bevorzugt sind textile Träger aus den vorgenannten Garnen die eine Dehnung in Längsrichtung von 10 bis 100 % und/oder in Querrichtung von 20 bis 300 % aufweisen.
2. Glasfasergewebe, -gewirke oder -gestricke eines Flächengewichts von 60 bis 500 g/m$^2$, vorzugsweise 100 bis 400 g/m$^2$, hergestellt aus Glasfasergarnen mit einem E-Modul von 7.000 bis 9.000 (daN/mm$^2$), und einer Fadenzahl von 3 bis 10, bevorzugt 5 bis 7 in Längsrichtung und mit einer Fadenzahl von 3 bis 10, bevorzugt 4 bis 6 in Querrichtung je Zentimeter Glasfasergewebe, die durch eine spezielle Art der Hitzebehandlung eine Längenelastizität von 10 bis 30 % besitzen, sind bevorzugt. Die Gewirke können sowohl geschlichtet als auch ungeschlichtet sein.
3. Nicht gebundene oder gebundene oder genadelte Vliese auf Basis von anorganischen und vorzugsweise organischen Fasern eines Flächengewichts von 30 bis 400 g/m$^2$, vorzugsweise 50 bis 200 g/m$^2$.

Für die Herstellung erfindungsgemäßer Konstruktionsmaterialien in Form von Schalen oder Schienen kommen auch Vliese mit Flächengewichten bis 1.000 g/m$^2$ in Betracht. Erfindungsgemäß geeignete Trägermaterialien werden beispielsweise auch in US 4 134 397, US 3 686 725, US 3 882 857, DE-A-3 211 634 und EP-A-61 642 beschrieben.

Bei den erfindungsgemäßen Konstruktionsmaterialien ist das Trägermaterial mit einer Menge von 25 bis 80 Gew.-%, bevorzugt von 30 bis 75 Gew.-%, an wasserhärtender Polymerzubereitung, bezogen auf das gesamte Material, beschichtet und/oder imprägniert.

Es wurde auch ein Verfahren zur Herstellung der erfindungsgemäßen Konstruktionsmaterialien gefunden, das dadurch gekennzeichnet ist, daß man ein wasserhärtendes Reaktivharz mit einem Polyetherpolysiloxan-Polyurethan-Additiv der Formel

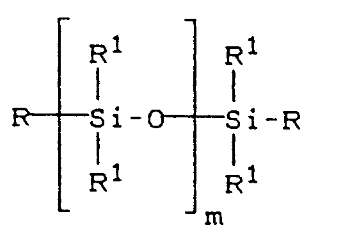

(I),

in der

$R^1$ für Niederalkyl steht,

m für die mittlere Zahl an Siloxangruppen im Bereich von 5 bis 25 steht,

R für den Rest

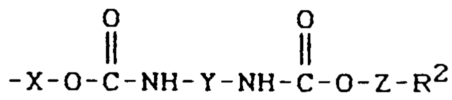

steht,

worin

X für einen Niederalkylenrest steht,

Y für einen aliphatischen, cycloaliphatischen, aromatischen oder araliphatischen Rest steht,

Z für einen Polyetherrest mit Ethylenoxid- und/oder Propylenoxid-Gruppen steht, wobei die mittlere Zahl der Ethylenoxid und/oder Propylenoxid-Gruppen im Bereich von 5 bis 150 liegt, und

$R^2$ für Niederalkyl steht,

wobei

Y durch weitere

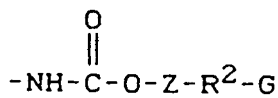 -Gruppen

substituiert sein kann,

in denen

Z und $R^2$ die obengenannte Bedeutung haben,

vermischt, Katalysatoren und weitere Hilfs- und Zusatzmittel zugibt und diese Mischung dann homogen über die Fläche des Trägermaterials verteilt.

Bei dem erfindungsgemäßen Verfahren arbeitet man unter Ausschluß von Feuchtigkeit. Bevorzugt wird bei einer relativen Feuchte <2 % (bei 21 °C), besonders bevorzugt bei <1 % (bei 21 °C), gearbeitet.

Für die Beschichtung bzw. Imprägnierung kann die Polymerzubereitung in einem inerten Lösungsmittel gelöst sein, das nach dem Beschichtungsvorgang wieder abgezogen wird.

Inerte Lösungsmittel können beispielsweise chlorierte Kohlenwasserstoffe, wie Methylenchlorid, Trichlorethan oder Chloroform, Ketone wie Aceton und Methylethylketon, Ester wie Essigsäureethylester und Butylacetat, Aromaten wie Toluol, Xylol oder entsprechende derivatisierte Typen, die keinen Zerewitinoff-aktiven Wasserstoff besitzen, sein.

Beispielsweise können die erfindungsgemäßen Konstruktionsmaterialien wie folgt hergestellt werden:
Im allgemeinen läßt man das Trägermaterial über eine Walze laufen und tränkt es mit der Polymerzubereitung, gegebenenfalls in einem Lösungsmittel. Unmittelbar nach der Beschichtung bzw. Imprägnierung wird das Material in der gewünschten Länge (in der Regel 2 bis 11 m) auf geeignete Spulen aufgerollt und in einer luft- und wasserdichten Folie (z.B. aus Kunststoff-Aluminium-Laminat) oder anderen völlig dichten Behältern versiegelt, wie sie in der DE-A-23 57 931, DE-A-26 51 089 und DE-A-30 33 569 beschrieben werden.

Unmittelbar vor der Anwendung wird das Material aus der Verpackung entnommen und um den betreffenden Gegenstand gewickelt oder anderweitig angebracht.

Für die Aushärtung werden die erfindungsgemäßen Zubereitungen mit Wasser, gegebenenfalls auch nur mit Luftfeuchtigkeit, in Kontakt gebracht.

Der Aushärtvorgang ist in der Regel kurz (etwa 3 bis 15 Min.). In dieser Zeit sind die erfindungsgemäßen Zubereitungen überraschenderweise gut modellierbar. Sie zeigen kaum Klebrigkeiten, ihr kinetischer Reibungskoeffizient liegt im allgemeinen unter 1,0.

Die erfindungsgemäßen Polymermodifikationen, die die Verarbeitung des beschichteten Trägermaterials wesentlich erleichtern, beeinflussen das Eigenschaftsbild der ausgehärteten Konstruktionsmaterialien in Bezug auf Härte, Bruchdehnung, Lagenverbund nicht.

Beispiel 1

Herstellung des erfindungsgemäßen Reaktivharz-Additivs

94,2 g Toluylen 2,4- und 2,6-diisocyanat-Gemisch im Isomerenverhältnis 80:20 werden in einer

Apparatur, bestehend aus einem Dreihalskolben mit Rührer, Tropftrichter und Rückflußkühler in 250 ml abs. Methylenchlorid vorgelegt.

Hierzu werden 1200 g eines monofunktionellen Polyethers, gestartet auf n-Butanol mit einem Mischblock aus 15 % Propylenoxid und 65 % Ethylenoxid und einem Endblock von 20 % Ethylenoxid (mittleres Molekulargewicht: 2440 g/mol), langsam unter Rückfluß des vorgelegten Gemisches zugetropft. Nach erfolgter Zugabe wird noch eine halbe Stunde unter Rückfluß erhitzt.

Dann werden 153 g eines bishydroxymethylfunktionellen Polydimethylsiloxans mit dem mittleren Molekulargewicht 566 g/mol zugegeben und das Reaktionsgemisch weitere 2 Stunden unter Rückfluß erhitzt. Zur Aufarbeitung wird das gesamte Reaktionsgemisch am Rotationsverdampfer vom Lösungsmittel befreit und ist nun als Additiv verwendbar.

Beispiel 2

Herstellung eines erfindungsgemäßen wasserhärtenden Reaktivharzes (unter Verwendung des erfindungsgemäßen Additivs)

In einem 10 l Sulfiergefäß mit Edelstahl-Ankerrührer werden 6,48 kg Isocyanat (Bis-(4-isocyanatophenyl)-methan, welches carbodiimidisierte Anteile enthält [NCO-Gehalt = 29 %]) vorgelegt. Dann werden 7,8 g eines Polydimethylsiloxans mit $\eta$ = 30.000 mPas und 4,9 g Benzoylchlorid sowie danach 1,932 kg eines durch Propoxylierung von Propylenglykol hergestellten Polyethers (OH-Zahl = 112 mg KOH/g), 1,29 kg eines durch Propylierung von Propylenglykol hergestellten Polyethers (OH-Zahl = 250 mg KOH/g) und 190 g Dimorpholinodiethylether zugegeben. Nach 30 Min. erreicht die Reaktionstemperatur 45°C, nach 1 Std. ist das Temperaturmaximum von 56°C erreicht, und der Isocyanatgehalt beträgt 14,2 %. Zu dem Reaktionsgemisch werden dann 500 g des in Beispiel 1 beschriebenen Additivs zugesetzt und homogen gerührt. Der Isocyanatgehalt beträgt am Ende 13,2 %, die Viskosität liegt bei 19 950 mPa.s

Beispiel 3

Herstellung eines wasserhärtenden Reaktivharzes (Vergleichsbeispiel ohne Additiv)

In einer Apparatur analog Beispiel 2 werden 6,48 kg Isocyanat (Bis(4-isocyanatophenyl)-urethan, welches carbodiimidisierte Anteile enthält [NCO-Gehalt = 29 %]) vorgelegt. Dann werden 7,8 g eines Polydimethylsiloxan mit $\eta_{25}$ = 30000 mPa.s und 4,9 g Benzoylchlorid sowie danach 1,93 kg eines durch Propoxylierung von Propylenglykol hergestellten Polyethers (OH-Zahl = 112 mg KOH/g), 1,29 kg eines durch Propoxylierung von Glycerin hergestellten Polyethers/OH-Zahl = 250 mg KOH/g) und 190 g Dimorpholinodiethylether zugegeben. Nach 30 Minuten erreicht die Reaktionstemperatur 42°C, nach 1 Std. ist das Temperaturmaximum von 48°C erreicht und der Isocyanatgehalt beträgt 13,6 %. Die Viskosität liegt bei 17800 mPa.s.

Beispiel 4

Herstellung eines erfindungsgemäßen wasserhärtenden Reaktivharzes (unter Verwendung des erfindungsgemäßen Additivs)

In einem 10 l Sulfiergefäß mit Edelstahl-Ankerrührer werden 6,5 kg Isocyanat (Bis-(4-isocyanatophenyl)-methan, welches carbodiimidisierte Anteile enthält [NCO-Gehalt = 29 %]), vorgelegt und auf ca. 50°C vorgewärmt. Dazu werden 150 g eines UV-Stabilisators ( ein Cyanalkylindolderivat) zugegeben und so lange gerührt, bis der gesamte Feststoff gelöst ist. Nach Abkühlen auf Raumtemperatur werden 3,5 kg propoxylierten Triethanolamins (OH-Zahl = 150 mg KOH/g) innerhalb von 10 Minuten zugesetzt. Nach kurzem Temperaturanstieg auf 55°C nach 55 Minuten fällt die Temperatur wieder und der Isocyanatgehalt erreicht nach 2 Stunden 13,4 %. Nach Zugabe von 534,2 g des in Beispiel 1 beschriebenen Additivs wird das Reaktionsgemisch homogen gerührt, seine Viskosität liegt bei 19 056 mPa.s (25°C).

Beispiel 5

Herstellung eines wasserhärtenden Reaktivharzes (Vergleichsbeispiel ohne Additiv)

In einem 10 l Sulfiergefäß mit Edelstahl-Ankerrührer werden 6,5 kg Isocyanat (Bis-(4-isocyanatophenyl)-methan, welches carbodiimidisierte Anteile enthält [NCO-Gehalt = 29 %]), vorgelegt und auf ca. 50 °C vorgewärmt. Dazu werden 150 g eines UV-Stabilisators ( ein Cyanalkylindolderivat) zugegeben und so lange gerührt, bis der gesamte Feststoff gelöst ist. Nach Abkühlen auf Raumtemperatur werden 3,5 kg propoxylierten Triethanolamins (OH-Zahl = 150 mg KOH/g) innerhalb von 10 Minuten zugesetzt. Nach kurzem Temperaturanstieg auf 55 °C nach 55 Minuten fällt die Temperatur wieder und der Isocyanatgehalt erreicht nach 2 Stunden 13,4 %. Der Isocyanatgehalt des fertigen Prepolymers beträgt 12,7 %, die Viskosität liegt bei 14.640 mPa.s (25 °C).

## Beispiel 6

### Herstellung von Probeverbänden mit den Reaktivharzen der Beispiele 2-5

6a) Ein Glasfasergemisch (Breite 10,0 cm, Quadratmetergewicht ca. 290 g/$m^2$), das in Längsrichtung eine Dehnung von ca. 65 % aufweist (eine ausführliche Beschreibung dieser Gewirke findet sich in der US 4 609 578), wird mit 80 Gew.-% (bezogen auf Gewirke) des Harzes gemäß Beispiel 2 beschichtet. Die Beschichtung erfolgt in einer Atmosphäre, deren relative Feuchte durch einen Taupunkt des Wassers von unterhalb -20 °C gekennzeichnet ist. Das Harz wird über ein geeignetes Walzenimprägniermittel homogen auf das Gewirke aufgebracht. Eine geeignete Apparatur ist in der US 4 427 002 detalliert beschrieben. Nach Beschichtung werden 3,66 m dieses Bandes auf einer Kunststoffspule von 1 cm Durchmesser aufgewickelt und in einer Wasserdampf undurchlässigen Folie versiegelt.

6b) Analog zu Beispiel (6a) wird das Glasfasergewirke mit 80 Gew.-% (bezogen auf Gewirke) des Harzes aus Beispiel 3 beschichtet und verpackt.

6c) Analog zu Beispiel (6a) wird das Glasfasergewirke mit 70 Gew.-% (bezogen auf Gewirke) des Harzes aus Beispiel 4 beschichtet und verpackt.

6d) Analog zu Beispiel (6a) wird das Glasfasergewirkt aus 70 Gew.-% (bezogen auf Gewirke) des Harzes mit Beispiel 5 beschichtet und verpackt.

6e) Analog zu Beispiel (6a) wird ein Polyestergewirke (Breite 10,0 cm, Quadratmetergewicht 118 g/$m^2$), das in Längsrichtung eine Dehnung von ca. 55 % und in Querrichtung eine Dehnung von ca. 90 % aufweist und welches im Maschenstab ein polyfiles, texturiertes Polyesterfilamentgarn (167 dtex, f 30x1) und in der Maschenreihe ein polyfiles hochfestes Polyesterfilamentgarn (550 dtex, f 96, normal schrumpfend) besitzt, mit 150 Gew.-% (bezogen auf Gewirke) des Harzes aus Beispiel 2 beschichtet und verpackt.

6f) Analog zu Beispiele (6a) wird das in Beispiel (6e) beschriebene Polyestergewirke mit 150 Gew.-% (bezogen auf Gewirke) des Harzes aus Beispiel 2 beschichtet und verpackt.

## Beispiel 7

### Bestimmung des kinetischen Reibungskoeffizienten der beschichteten Trägermaterialien 6a-6f

In völliger Analogie zur EP 221 669 wurde entsprechend dem ASTm D-1894-Test mit einer ApReibungskoeffizienttron Corp. (Instron Coefficient of Friction Fixture; Catalog N. 2810-005) und dem in der EP-A 221 669, Seite 13, beschriebenen Edelstahlschlitten der kinetische Reibungskoeffizient bestimmt.

Die Probeverbände wurden nach 1 Monat der Verpackung entnommen und wie in EP-A 221 669 Seite 14, 15, beschrieben aufbereitet und vermessen. Die Kraftmessung erfolgte mit einer ZWICK Universal Prüfmaschine Typ 1484.

| Vergleichsproben: | Kin. Reibungskoeffizient |
|---|---|
| Scotchcast® (ohne Gleitmittel, s. EP 221 669, Seite 17) | 2,0 |
| Scotchcast® Plus (mit Gleitmittel, Ethylenoxid im Reaktivharz, 4,7 % Polydimethylsiloxan-Zusatz) | 0,3 |

| Beispiele (erfindungsgemäß) | Kin. Reibungskoeffizient |
|---|---|
| 6a | 0,8 |
| 6b | 1,5 |
| 6c | 0,7 |
| 6d | 1,6 |
| 6e | 0,3 |
| 6f | 0,8 |

## Patentansprüche

1. Konstruktionsmaterial, bestehend aus einer Trägerschicht, die mit einer wasserhärtenden Polymerzubereitung beschichtet und/oder imprägniert ist, wobei die Polymerzubereitung als Additiv Polyether-polysiloxan-Polyurethane der Formel

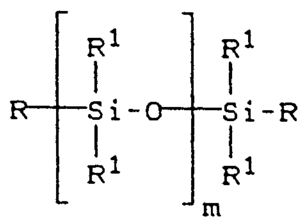
,

in der

| | |
|---|---|
| $R^1$ | für $C_1$-$C_6$-Alkyl steht, |
| m | für die mittlere Zahl an Siloxangruppen im Bereich von 5 bis 25 steht, |
| R | für den Rest |

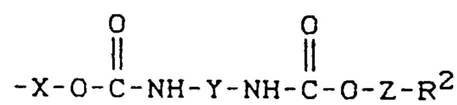

| | |
|---|---|
| | steht, worin |
| X | für $C_1$-$C_6$-Alkylen steht, |
| Y | für einen aliphatischen, cycloaliphatischen, aromatischen oder araliphatischen Rest steht, |
| Z | für einen Polyetherrest mit Ethylenoxid und/ oder Propylenoxid-Gruppen steht, wobei die mittlere Zahl der Ethylenoxid und/oder Propylenoxid-Gruppen im Bereich von 10 bis 100 liegt, und |
| $R^2$ | für $C_1$-$C_6$-Alkyl steht, wobei |
| Y | durch weitere |

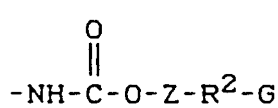-Gruppen

| | |
|---|---|
| | substituiert sein kann, in denen |
| Z und $R^2$ | die obengenannte Bedeutung haben, |

enthält.

**2.** Konstruktionsmaterialien nach Anspruch 1, enthaltend Polyetherpolysiloxan-Polyurethane, worin

| | |
|---|---|
| $R^1$ | für Methyl oder Ethyl steht, |
| m | für die mittlere Zahl an Siloxangruppen im Bereich von 5 bis 25 steht, |
| R | für den Rest |

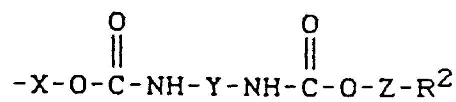

steht,
worin

| | |
|---|---|
| X | für Alkylen ($C_1$ bis $C_4$) steht, |
| Y | einen aliphatischen Kohlenwasserstoffrest mit 2 bis 18 Kohlenstoffatomen, einen cycloaliphatischen Kohlenwasserstoffrest mit 4 bis 15 Kohlenstoffatomen, einen aromatischen Kohlenwasserstoffrest mit 6 bis 15 Kohlenstoffatomen oder einen araliphatischen Kohlenwasserstoffrest mit 8 bis 15 Kohlenstoffatomen bedeutet, |
| Z | für einen Polyetherrest mit Ethylenoxid- und Propylenoxid-Gruppen steht, wobei die mittlere Zahl der Ethylenoxid- und Propylenoxid-Gruppen im Bereich von 5 bis 150 liegt, und |
| $R^2$ | für Alkyl ($C_1$ bis $C_4$) steht, |

wobei das Gewichts-Verhältnis der Ethylenoxid-zu Propylenoxid-Gruppen 10:90 % bis 80:20 % beträgt und
wobei Y durch 1 oder 2 weitere

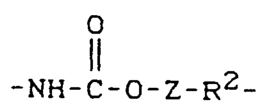 -Gruppen

substituiert sein kann,
in denen

| | |
|---|---|
| Z und $R^2$ | die obengenannte Bedeutung haben. |

**3.** Konstruktionsmaterialien nach den Ansprüchen 1 und 2, enthaltend Polyetherpolysiloxan-Polyurethane worin

| | |
|---|---|
| $R^1$ | für Methyl oder Ethyl steht, |
| m | für die mittlere Zahl an Siloxangruppen im Bereich von 5 bis 25 steht, |
| R | für den Rest |

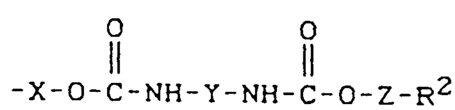

steht,
worin

| | |
|---|---|
| X | für Alkylen ($C_1$ bis $C_4$) steht, |
| Y | einen aliphatischen Kohlenwasserstoffrest mit 6 bis 10 Kohlenstoffatomen, einen cycloaliphatischen Kohlenwasserstoffrest mit 5 bis 10 Kohlenstoffatomen, einen aromatischen Kohlenwasserstoffrest mit 6 bis 13 Kohlenstoffatomen oder einen araliphatischen Kohlenwasserstoffrest mit 8 bis 13 Kohlenstoffatomen bedeutet, |

| | |
|---|---|
| Z | für einen Polyetherrest mit Ethylenoxid- und Propylenoxid-Gruppen steht, wobei die mittlere Zahl der Ethylenoxid- und Propylenoxid-Gruppen im Bereich von 5 bis 100 liegt, und |
| $R^2$ | für Alkyl ($C_1$ bis $C_4$) steht, wobei das Gewichts-Verhältnis der Ethylenoxid-zu Propylenoxid-Gruppen 10:90 % bis 80:20 % beträgt und wobei Y durch eine weitere |

$$-NH-\overset{\overset{\displaystyle O}{\|}}{C}-O-Z-R^2\text{-Gruppe}$$

substituiert sein kann,
in der

| | |
|---|---|
| Z und $R^2$ | die obengenannte Bedeutung haben. |

**4.** Konstruktionsmaterialien nach den Ansprüchen 1 bis 3, enthaltend 0,1 bis 10 Gew.-% Polyetherpolysiloxan-Polyurethane bezogen auf das wasserhärtende Polymer.

**5.** Verfahren zur Herstellung von Konstruktionsmaterialien, dadurch gekennzeichnet, daß man ein wasserhärtendes Reaktivharz mit einem Polyetherpolysiloxan-Polyurethan-Additiv der Formel

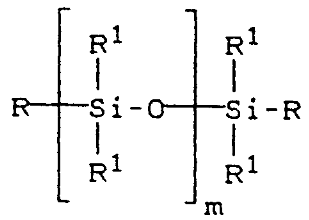
,

in der

| | |
|---|---|
| $R^1$ | für $C_1$-$C_6$-Alkyl steht, |
| m | für die mittlere Zahl an Siloxangruppen im Bereich von 5 bis 25 steht, |
| R | für den Rest |

$$-X-O-\overset{\overset{\displaystyle O}{\|}}{C}-NH-Y-NH-\overset{\overset{\displaystyle O}{\|}}{C}-O-Z-R^2$$

steht,
worin

| | |
|---|---|
| X | für $C_1$-$C_6$-Alkylen steht, |
| Y | für einen aliphatischen, cycloaliphatischen, aromatischen oder araliphatischen Rest steht, |
| Z | für einen Polyetherrest mit Ethylenoxid- und/ oder Propylenoxid-Gruppen steht, wobei die mittlere Zahl der Ethylenoxid und/oder Propylenoxid-Gruppen im Bereich von 5 bis 150 liegt, und |
| $R^2$ | für $C_1$-$C_6$-Alkyl steht, wobei |
| Y | durch weitere |

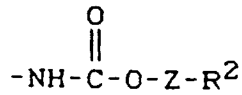 -Gruppen

substituiert sein kann,
in denen
Z und $R^2$ die obengenannte Bedeutung haben,
vermischt, Katalysatoren und weitere Hilfs-und Zusatzmittel zugibt und dann homogen über die Fläche des Trägermaterials verteilt.

**6.** Verwendung von wasserhärtenden Polymerzubereitungen enthaltend ein Polyetherpolysiloxan-Polyurethan-Additiv der Formel

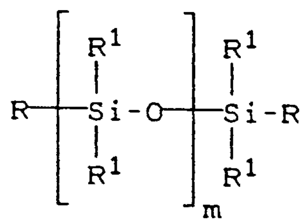 ,

in der
$R^1$ für $C_1$-$C_6$-Alkyl steht,
m für die mittlere Zahl an Siloxangruppen im Bereich von 5 bis 25 steht,
R für den Rest

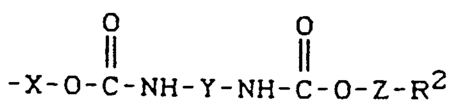

steht,
worin
X für $C_1$-$C_6$-Alkylen steht,
Y für einen aliphatischen, cycloaliphatischen, aromatischen oder araliphatischen Rest steht,
Z für einen Polyetherrest mit Ethylenoxid- und/ oder Propylenoxid-Gruppen steht, wobei die mittlere Zahl der Ethylenoxid und/oder Propylenoxid-Gruppen im Bereich von 5 bis 150 liegt, und
$R^2$ für $C_1$-$C_6$-Alkyl steht,
wobei
Y durch weitere

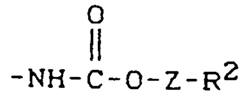 -Gruppen

substituiert sein kann,
in denen
Z und $R^2$ die obengenannte Bedeutung haben, für Konstruktionsmaterialien.

**7.** Verwendung nach Anspruch 6, dadurch gekennzeichnet, daß als wasserhärtende Polymerzubereitung Polyisocyanate bzw. entsprechende Prepolymere mit mehr als zwei Isocyanatgruppen eingesetzt

werden.

8. Verwendung der Zubereitungen nach den Ansprüchen 6 und 7 zur Herstellung von medizinischen Stützverbänden.

9. Verwendung der Zubereitungen nach den Ansprüchen 6 und 7 zur Herstellung von technischen Konstruktionsmaterialien.

**Claims**

1. Construction material consisting of a carrier layer which is coated and/or impregnated with a water-hardening polymer preparation, the polymer preparation containing as additive polyether polysiloxane polyurethanes of the formula

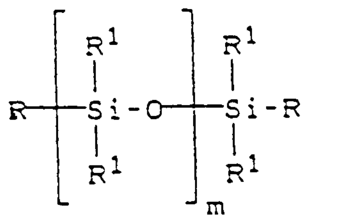

,

in which
$R^1$ represents $C_1$-$C_6$-alkyl,
m represents the average number of siloxane groups in the range from 5 to 25,
R represents the radical

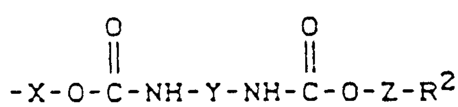

in which
X represents $C_1$-$C_6$-alkylene,
Y represents an aliphatic, cycloaliphatic, aromatic or araliphatic radical,
Z represents a polyether radical having ethylene oxide and/or propylene oxide groups, the average number of ethylene oxide and/or propylene oxide groups being in the range from 10 to 100 and
$R^2$ represents $C_1$-$C_6$-alkyl,
it being possible for
Y to be substituted by further

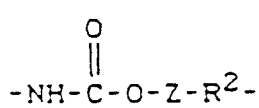

groups in which
Z and $R^2$ have the abovementioned meaning.

2. Construction materials according to Claim 1, containing polyether polysiloxane polyurethanes, in which
$R^1$ represents methyl or ethyl,
m represents the average number of siloxane groups in the range from 5 to 25,
R represents the radical

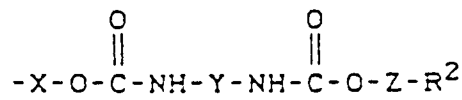

in which

X represents alkylene ($C_1$ to $C_4$),

Y represents an aliphatic hydrocarbon radical having 2 to 18 carbon atoms, a cycloaliphatic hydrocarbon radical having 4 to 15 carbon atoms, an aromatic hydrocarbon radical having 6 to 15 carbon atoms or an araliphatic hydrocarbon radical having 8 to 15 carbon atoms,

Z represents a polyether radical having ethylene oxide and propylene oxide groups, the average number of ethylene oxide and propylene oxide groups being in the range from 5 to 150, and

$R^2$ represents alkyl ($C_1$ to $C_4$), the weight ratio of ethylene oxide groups to propylene oxide groups being 10:90 % to 80:20 %, and it being possible for

Y to be substituted by 1 or 2 further

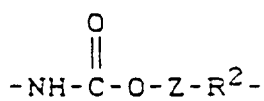

groups in which

Z and $R^2$ have the abovementioned meaning.

**3.** Construction materials according to Claims 1 and 2, containing polyether polysiloxane polyurethanes in which

$R^1$ represents methyl or ethyl,

m represents the average number of siloxane groups in the range from 5 to 25,

R represents the radical

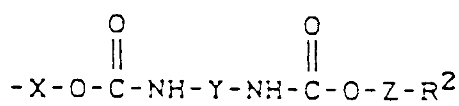

in which

X represents alkylene ($C_1$ to $C_4$),

Y represents an aliphatic hydrocarbon radical having 6 to 10 carbon atoms, a cycloaliphatic hydrocarbon radical having 5 to 10 carbon atoms, an aromatic hydrocarbon radical having 6 to 13 carbon atoms or an araliphatic hydrocarbon radical having 8 to 13 carbon atoms,

Z represents a polyether radical having ethylene oxide and propylene oxide groups, the average number of ethylene oxide and propylene oxide groups being in the range from 5 to 100 and

$R^2$ represents alkyl ($C_1$ to $C_4$), the weight ratio of ethylene oxide groups to propylene oxide groups being 10:90 % to 80:20 %, and it being possible for

Y to be substituted by a further

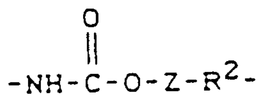

group in which

Z and $R^2$ have the abovementioned meaning.

**4.** Construction materials according to Claims 1 to 3, containing 0.1 to 10% by weight of polyether polysiloxane polyurethanes, based on the water-hardening polymer.

**5.** Process for the production of construction materials, characterised in that a water-hardening reactive resin is mixed with a polyether polysiloxane polyurethane additive of the formula

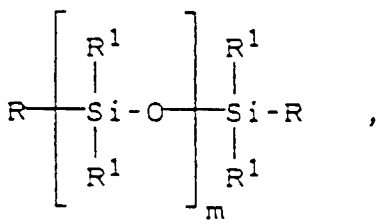

in which

$R^1$ represents $C_1$-$C_6$-alkyl,

m represents the average number of siloxane groups in the range from 5 to 25,

R represents the radical

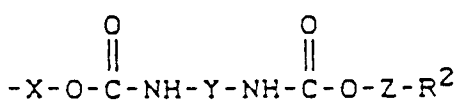

in which

X represents $C_1$-$C_6$-alkylene,

Y represents an aliphatic, cycloaliphatic, aromatic or araliphatic radical,

Z represents a polyether radical having ethylene oxide and/or propylene oxide groups, the average number of ethylene oxide and/or propylene oxide groups being in the range from 5 to 150, and

$R^2$ represents $C_1$-$C_6$-alkyl,

it being possible for

Y to be substituted by further

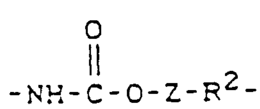

groups in which

Z and $R^2$ have the abovementioned meaning,

catalysts and further auxiliaries and additives are added and the mixture is then distributed homogeneously over the surface of the carrier material.

**6.** Use of water-hardening polymer preparations containing a polyether polysiloxane polyurethane additive of the formula

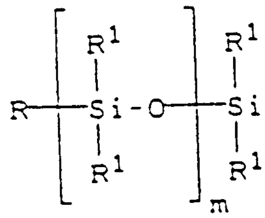 ,

in which

| | |
|---|---|
| $R^1$ | represents $C_1$-$C_6$-alkyl, |
| m | represents the average number of siloxane groups in the range from 5 to 25, |
| R | represents the radical |

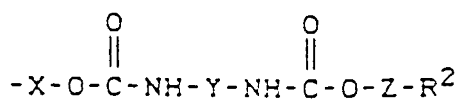

| | |
|---|---|
| | in which |
| X | represents $C_1$-$C_6$-alkylene, |
| Y | represents an aliphatic, cycloaliphatic, aromatic or araliphatic radical, |
| Z | represents a polyether radical having ethylene oxide and/or propylene oxide groups, the average number of ethylene oxide and/or propylene oxide groups being in the range from 5 to 150, and |
| $R^2$ | represents $C_1$-$C_6$-alkyl, it being possible for |
| Y | to be substituted by further |

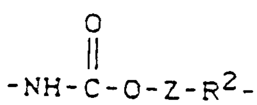

| | |
|---|---|
| | groups, in which |
| Z and $R^2$ | have the abovementioned meaning, for construction materials. |

**7.** Use according to Claim 6, characterised in that the water-hardening polymer preparation used comprises polyisocyanates or corresponding prepolymers having more than two isocyanate groups.

**8.** Use of the preparations according to Claims 6 and 7 for the production of medical support dressings.

**9.** Use of the preparations according to Claims 6 and 7 for the production of industrial construction materials.

**Revendications**

**1.** Matériau consistant en une couche de support revêtue et/ou imprégnée d'une composition polymère durcissant à l'eau, la composition polymère contenant en tant qu'additif un polyéther-polysiloxane-polyuréthanne de formule

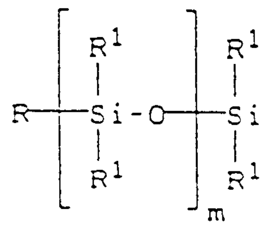 ,

dans laquelle

$R^1$ représente un groupe alkyle en $C_1$-$C_6$,

n est le nombre moyen des groupes siloxane qui se situe dans l'intervalle de 5 à 25,

R représente le groupe

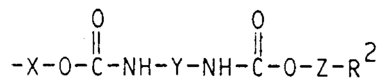

dans lequel

X représente un groupe alkylène en $C_1$-$C_6$,

Y représente un radical aliphatique, cycloaliphatique, aromatique ou araliphatique,

Z représente un radical de polyéther à groupes oxyde d'éthylène et/ou oxyde de propylène, le nombre moyen des groupes oxyde d'éthylène et/ou oxyde de propylène se situant dans l'intervalle de 10 à 100 et

$R^2$ représente un groupe alkyle en $C_1$-$C_6$,

Y pouvant porter d'autres substituants

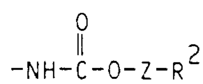

dans lesquels Z et $R^2$ ont les significations indiquées ci-dessus.

**2.** Matériaux selon la revendication 1, contenant des polyéther-polysiloxane-polyuréthannes dans lesquels

$R^1$ représente un groupe méthyle ou éthyle.

m est le nombre moyen des groupes siloxane qui se situe dans l'intervalle de 5 à 25,

R représente le groupe

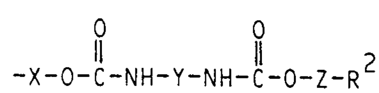

dans lequel

X représente un groupe alkylène en $C_1$-$C_4$,

Y représente
un radical hydrocarboné aliphatique en $C_2$-$C_{18}$,
un radical hydrocarboné cycloaliphatique en $C_4$-$C_{15}$,
un radical hydrocarboné aromatique en $C_6$-$C_{15}$ ou bien
un radical hydrocarboné araliphatique en $C_8$-$C_{15}$,

Z représente un radical de polyéther à groupes oxyde d'éthylène et oxyde de propylène, le nombre moyen des groupes oxyde d'éthylène et oxyde de propylène se situant dans l'intervalle de 5 à 150 et

$R^2$ représente un groupe alkyle en $C_1$-$C_4$,

les proportions relatives en poids entre les groupes oxydes d'éthylène et oxyde de propylène allant de 10 : 90 % à 80 : 20 % et

Y peut porter un ou deux autres substituants

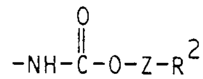

dans lesquels Z et $R^2$ ont les significations indiquées ci-dessus.

**3.** Matériaux selon les revendications 1 et 2, contenant des polyéther-polysiloxane-polyuréthannes dans lesquels

R¹ représente un groupe méthyle ou éthyle,

m est le nombre moyen des groupes siloxane qui se situe dans l'intervalle de 5 à 25,

R représente le groupe

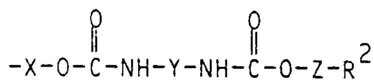

dans lequel

X représente un groupe alkylène en $C_1$-$C_4$,

Y représente
un radical hydrocarboné aliphatique en $C_6$-$C_{10}$,
un radical hydrocarboné cycloaliphatique en $C_5$-$C_{10}$,
un radical hydrocarboné aromatique en $C_6$-$C_{13}$ ou
un radical hydrocarboné araliphatique en $C_8$-$C_{13}$,

Z représente un radical de polyéther à groupes oxyde d'éthylène et oxyde de propylène, le nombre moyen des groupes oxyde d'éthylène et oxyde de propylène se situant dans l'intervalle de 5 à 100 et

$R^2$ représente un groupe alkyle en $C_1$-$C_4$,
les proportions relatives en poids entre les groupes oxyde d'éthylène et oxyde de propylène allant de 10 : 90 % à 80 : 20 % et

Y peut porter un autre substituant

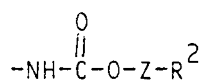

dans lequel Z et $R^2$ ont les significations indiquées ci-dessus.

**4.** Matériaux selon les revendications 1 à 3, contenant 0,1 à 10 % en poids de polyéther-polysiloxane-polyurathannes par rapport au polymère durcissant à l'eau.

**5.** Procédé de préparation de matériaux, caractérisé en ce que l'on mélange une résine réactive durcissant à l'eau avec un additif du type polyéther-polysiloxane-polyuréthanne de formule

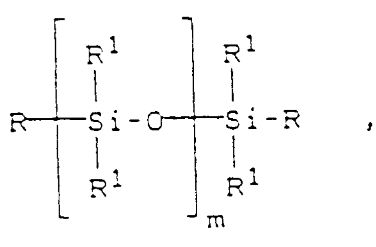

dans laquelle

$R^1$ représente un groupe alkyle en $C_1$-$C_6$,

m est le nombre moyen des groupes siloxane qui se situe dans l'intervalle de 5 à 25,
R représente le groupe

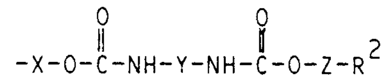

dans lequel
X représente un groupe alkylène en $C_1$-$C_6$,
Y représente un radical aliphatique, cycloaliphatique, aromatique ou araliphatique,
Z représente un radical de polyéther à groupes oxyde d'éthylène et/ou oxyde de propylène, le nombre moyen des groupes oxyde d'éthylène et/ou oxyde de propylène se situant dans l'intervalle de 5 à 150 et
$R^2$ représente un groupe alkyle en $C_1$-$C_6$,
Y pouvant porter d'autres substituants

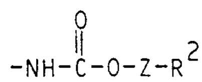

dans lesquels Z et $R^2$ ont les significations indiquées ci-dessus,
on ajoute de, catalyseurs et d'autres produits auxiliaires et additif puis on répartit de manière homogène sur la surface de la matière de support.

**6.** Utilisation de compositions polymères durcissant à l'eau et contenant en tant qu'additif un polyéther-polysiloxane-polyuréthanne de formule

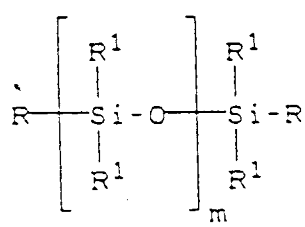 ,

dans laquelle
$R^1$ représente un groupe alkyle en $C_1$-$C_6$,
m est le nombre moyen des groupes siloxane qui se situe dans l'intervalle de 5 à 25,
R représente le groupe

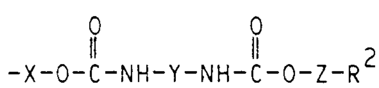

dans lequel
X représente un groupe alkylène en $C_1$-$C_6$,
Y représente un radical aliphatique, cycloaliphatique, aromatique ou araliphatique,
Z représente un radical de polyéther à groupes oxyde d'éthylène et/ou oxyde de propylène, le nombre moyen des groupes oxyde d'éthylène et/ou oxyde de propylène se situant dans l'intervalle de 5 à 150 et
$R^2$ représente un groupe alkyle en $C_1$-$C_6$,
Y pouvant porter d'autres substituants

$$-NH-\overset{\overset{\displaystyle O}{\|}}{C}-O-Z-R^{2}$$

dans lesquels Z et $R^2$ ont les significations indiquées ci-dessus, pour des matériaux.

**7.** Utilisation selon la revendication 6, caractérisée en ce que l'on utilise en tant que compositions polymères durcissant à l'eau des polyisocyanates ou leurs prépolymères contenant plus de deux groupes isocyanate.

**8.** Utilisation des compositions selon les revendications 6 et 7 pour la confection de bandages de support médicaux.

**9.** Utilisation des compositions selon les revendications 6 et 7 pour la fabrication de matériaux de construction techniques.